# DEMANDE DE BREVET EUROPEEN

(11) **EP 3 757 567 A1**
(43) Date de publication de la demande: **30.12.2020**
(21) Numéro de dépôt: 20180847.4
(22) Date de dépôt: 18.06.2020
(51) Int. Cl.: G01N 33/00, G01N 15/06, F24F 110/64

(54) **PROCÉDÉ DE MESURE DE LA POLLUTION PARTICULAIRE DANS UN ENVIRONNEMENT, PROGRAMME D'ORDINATEUR ET SUPPORT D'ENREGISTREMENT CORRESPONDANTS**

(30) Priorité: 24.06.2019 FR 1906818
(71) Demandeur: Electricité de France, 75008 Paris (FR); CENTRE NATIONAL DE LA RECHERCHE SCIENTIFIQUE -CNRS-, 75016 Paris (FR); Universite De La Rochelle, 17000 La Rochelle (FR)
(72) Inventeur: PICARD, Charles-Florian, 94120 FONTENAY SOUS BOIS (FR); WALL-RIBOT, Bénédicte, 91100 VILLABE (FR); DUFORESTEL, Thierry, 77250 MORET SUR LOING (FR); LIMAM, Karim, 17140 LAGORD (FR); ABADIE, Marc, 17000 LA ROCHELLE (FR)
(74) Mandataire: Regimbeau

(57) **Abrégé**

La présente invention se rapporte notamment à un procédé de mesure en continu de la pollution particulaire dans un environnement déterminé, dit "environnement suivi" (Esui), caractérisé par le fait qu'il comprend les étapes qui consistent à :
a/ disposer dans un environnement de référence (Eref) qui est du même type de ledit environnement suivi (Esui), d'une part un appareil de référence (AR) de mesure de pollution particulaire et d'autre part d'une "nappe" (N), c'est à dire un ensemble d'au moins deux autres appareils (A1-A5) de mesure de pollution particulaire, les appareils (A1-A5) de ladite nappe (N) étant différents les uns des autres, mais également dudit appareil de référence (AR) ;
b/ pendant une durée prédéterminée et à plusieurs instants t pendant cette durée, collecter les mesures réalisées par la totalité de ces appareils (A1-A5) et constituer une base de données de l'ensemble de ces mesures ;
c/ établir, uniquement à partir des mesures de ladite base de données, une loi mathématique reliant la mesure réalisée par ledit appareil de référence (AR) à celles de ladite nappe (N) d'appareils ;
d/ disposer, dans ledit environnement suivi (Esui), seulement la nappe (N) d'appareils, et procéder à des mesures à l'aide de cette nappe (N) pour prédire, en faisant usage de ladite loi mathématique, les mesures que donnerait ledit appareil de référence (AR) s'il était dans cet environnement suivi (Esui).

## Description

### DOMAINE DE L'INVENTION

La présente invention se situe dans le domaine de la mesure de la pollution particulaire dans un environnement.

### ARRIERE PLAN TECHNOLOGIQUE DE L'INVENTION

Le contexte contraint de maîtrise des consommations énergétiques des bâtiments a poussé les entreprises à la conception et la réhabilitation de bâtiments très performants énergétiquement reposant principalement sur l'utilisation des énergies renouvelables, mais oubliant très souvent le rôle prépondérant des occupants des bâtiments en n'intégrant pas des critères tels quel le confort global, le bien-être et la qualité de l'air (impact sanitaire).

La prise en compte de ces critères et plus spécifiquement celui relatif à la qualité de l'air de bâtiments ("QAI" pour "Qualité de l'Air Intérieur) constitue un enjeu important pour la conception et à la rénovation des bâtiments énergétiquement performants pour les prochaines années.

La quantification de la qualité de l'air implique la mesure dynamique de faibles concentrations de polluants de nature distincte : physique (humidité, température, dioxyde de carbone), chimique, particulaire, bactérienne, etc. Si la mesure en continue de certains paramètres de confort (température, humidité relative, etc.) peut s'effectuer facilement avec des appareils de mesures peu couteux et fiables tel que des micro-capteurs, ce n'est pas le cas pour tous les polluants comme les polluants de nature chimique ou particulaire. La mesure fiable, dynamique et à cout raisonnable (une centaine d'euros) des particules et en particulier des particules de très petites tailles (diamètre inférieur au micromètre) ayant les impacts sanitaires les plus importants est actuellement impossible.

Pour mesurer en continu et de manière dynamique les concentrations de particules de différentes tailles dans l'air, il existe deux catégories d'appareils de mesure plus ou moins mobiles : ceux que l'on peut qualifier d'appareils de qualité de laboratoire (couteux et à l'utilisation généralement contraignante) et ceux qui appartiennent plutôt à une famille des capteurs dits " à bas cout", qui sont qualifiés de "micro-capteurs".

Ces derniers se sont développés au début des années 2000. Le marché de ces micro-capteurs est en pleine expansion et est lié à l'intérêt conjugué des personnes à la fois pour les objets connectés et pour les impacts sur la santé de l'air respiré. Leur utilisation offre de nouvelles perspectives dans la gestion de la QAI et leur déploiement à grande échelle permet de répondre au besoin croissant de maîtrise de sa qualité d'air intérieur.

La mesure en continu de particules dans l'air repose principalement sur trois grandes techniques :
- La mesure de la masse (pesée directe ou indirecte) des particules déposées sur un collecteur (filtre, tube de verre effilé...) ;
- La mesure optique (diffusion d'un faisceau lumineux par les particules) ;
- L'atténuation d'un rayonnement béta (électrons) par une matière solide (dépendante uniquement de sa masse et non d'autres paramètres tels que sa densité, forme ou composition).

Ainsi, en ce qui concerne la mesure dynamique par pesée, un principe utilisé consiste, par exemple, à mesurer la modification de la fréquence de résonnance d'un matériau selon la masse des particules déposée à sa surface. Ce principe est utilisé par les appareils de type TEOM ("Tapered Element Oscillating Microbalance") et plus récemment par des micro-capteurs en cours de développement dans des laboratoires.

Ce type de mesure permet d'obtenir directement les indicateurs recherchés de type PM1, PM2.5 et PM10, c'est à dire la masse cumulée des particules de diamètres inférieurs à respectivement 1, 2.5 et 10 micromètres. La mesure est précise pour les appareils de type TEOM. Pour les autres, la précision reste à définir. L'inconvénient majeur des appareils de type TEOM est leur cout (rédhibitoire) et leur encombrement (non portable). Ils peuvent en outre nécessiter l'adjonction de modules pour prendre en compte les composés semi-volatiles.

Pour la mesure dynamique optique, le principe repose sur la détection d'un rayonnement diffusé par la présence de particules dans une certaine direction (Théorie de Mie). Le signal obtenu varie en fonction de la taille et de la nature des particules, ce qui permet de les quantifier et les qualifier par leur taille. Plusieurs appareils de mesure de particules utilisent ce principe qui est utilisé à la fois par les micro-capteurs de particules (faible coût) et des appareils de qualité laboratoire (coût très élevé). Tous ces appareils sont portables et permettent une mesure dynamique des concentrations des particules.

Enfin, pour ce qui est de la mesure par atténuation d'un rayonnement béta, cette atténuation par une matière solide dépend uniquement de sa masse et non d'autres paramètres tels que sa densité, forme ou composition. Ces appareils sont onéreux et, bien que mobiles, gardent une portabilité limitée.

Les micro-capteurs commercialisés jusqu'à présent permettant de mesurer en continu et de manière dynamique les concentrations des particules dans l'air reposent pour la très grande majorité des cas sur la détection d'un rayonnement diffusé dans une certaine direction. Ils présentent l'avantage d'être de faible encombrement et donc de pouvoir être introduits soit dans des dispositifs de mesure portables soit directement dans des systèmes ou équipements intégrés ou faisant partie des bâtiments.

Malgré leurs avantages et les nombreuses opportunités scientifiques envisagées, ils présentent l'inconvénient majeur de ne pas être suffisamment fiables et de ne pas détecter toutes les tailles de particules (notamment les plus fines, de diamètre inférieur à 0,3 µm), caractéristique qui est intrinsèque au principe physique de mesure mis en œuvre, à savoir la détection de rayons sous un angle donné (Théorie de Mie).

Un graphique de l'intensité diffusée relative en fonction du diamètre des particules montre par exemple que, à un angle donné et à une intensité mesurée, plusieurs tailles de particules sont possibles.

En outre, les particules présentes dans l'air sont très variées à la fois en nature et en forme. Les calculs de la théorie de Mie (particules sphériques homogènes) ne sont donc pas directement applicables. Il en découle que cette technique présente des limites pour la mesure des particules dans l'air et une faible fiabilité de la mesure des particules par les micro-capteurs optiques.

Pour limiter ce problème, plusieurs solutions ont été proposées. Une solution consiste à introduire des facteurs dits de calibration pour chaque type de particules. Ces facteurs permettent alors de corriger le signal délivré par les micro-capteurs en les adaptant au type de pollution particulaire. Bien qu'intéressante, cette approche s'avère vite complexe à mettre en œuvre dans le domaine de la mesure des particules, compte tenu de la très grande variété des particules présentes autant sur leur distribution granulométrique que sur leur forme et leur nature. Et comme l'ont montré Li et Biswas (2017), même pour des particules présentant une répartition granulométrique voisine mais de nature différente comme pour les particules de Nacl et de sucralose, les facteurs peuvent varier d'un rapport allant de 1 à 5. Cette approche est adaptée lorsqu'un type de particules est prédominant, comme cela peut être le cas pour des poussières industrielles lié à un processus spécifique.

Une seconde voie a également été proposée. Celle-ci consiste à utiliser simultanément plusieurs longueurs d'ondes et plusieurs angles de détection au sein d'un même capteur pour corriger l'influence d'un interférant généralement l'humidité et donc améliorer la qualité des mesures (Wenjia Shao, 2017). Ainsi, le document FR3066599 repose aussi sur ce principe d'utilisation d'un capteur intégrant deux angles de déviation d'un rayon lumineux.

En tout état de cause, il demeure un problème technique non complètement résolu à ce jour de pouvoir disposer d'un système de mesure continue et dynamique de particules dans l'air qui fasse usage de micro-capteurs à bas coût et dont la qualité de l'information se rapprocherait de celle fournie par un appareil de laboratoire, à la fois au niveau de la précision/justesse de ses sorties mais aussi au niveau largeur du profil granulométrique appréhendé de la pollution étudiée.

On décrit par ailleurs dans US2018/0149577 un dispositif générant des données de comptage de particules et/ou de distribution granulométriques des particules à partir de dispositifs peu coûteux.

Ces données sur le comptage et/ou la distribution granulométrique des particules sont ensuite converties en une mesure de la concentration massique de particules à l'aide d'un modèle de conversion élaboré à partir des mesures de référence de la concentration massique de particules générées par des instruments de concentration massique de particules à coût élevé.

Le modèle de conversion est initialement développé sur la base d'une comparaison des données d'un capteur optique de référence avec les données d'une station de surveillance. Des capteurs de paramètres environnementaux sont disposés au même endroit que l'appareil de laboratoire et que l'appareil à bas cout dit de référence (c'est-à-dire celui qui sert à établir la loi). Les autres capteurs à bas cout de particules du réseau sont distants. Ainsi, on établit une loi entre un appareil à bas coût et un appareil de laboratoire, dans les conditions météorologiques du moment et on applique cette loi aux autres capteurs de particules distants. Cette technique a donc pour vocation de calibrer les capteurs à bas coût (ou pas) à partir d'un capteur de référence et de capteurs "météorologiques". Cette loi de calibration est appliquée aux autres capteurs à bas cout du réseau qui sont géographiquement distants.

Par ailleurs, on décrit dans US2019/0137363 un système de mesure des émissions prévu pour fournir une estimation précise et en temps réel du nombre de particules (PN)/ de matières particulaires (PM) dans les gaz d'échappement. Le système est capable de différencier avec précision la taille et la composition des PM / PN en synchronisant des capteurs configurés de manière différente. L'échappement peut être généré par un moteur à combustion interne, et dans ce cas le système peut être connecté séquentiellement à l'échappement du moteur à combustion interne.

### RESUME DE L'INVENTION

Le problème technique formulé plus haut est résolu selon l'invention par le présent procédé de mesure en continu de la pollution particulaire dans un environnement déterminé, dit "environnement suivi", caractérisé par le fait qu'il comprend les étapes qui consistent à :
a/ disposer dans un environnement de référence qui est du même type de ledit environnement suivi, d'une part un appareil de référence de mesure de pollution particulaire et d'autre part d'une "nappe", c'est à dire un ensemble d'au moins deux autres appareils de mesure de pollution particulaire, les appareils de ladite nappe (N) étant différents les uns des autres, mais également dudit appareil de référence ;
b/ pendant une durée prédéterminée et à plusieurs instants t pendant cette durée, collecter les mesures réalisées par la totalité de ces appareils et constituer une base de données de l'ensemble de ces mesures ;
c/ établir, uniquement à partir des mesures de ladite base de données, une loi mathématique reliant la mesure réalisée par ledit appareil de référence à celles de ladite nappe d'appareils ;
d/ disposer, dans ledit environnement suivi, seulement la nappe d'appareils, et procéder à des mesures à l'aide de cette nappe pour prédire, en faisant usage de ladite loi mathématique, les mesures que donnerait ledit appareil de référence s'il était dans cet environnement suivi.

Selon d'autres caractéristiques non limitatives et avantageuses de l'invention :
- ledit environnement suivi est un espace ouvert ou un espace fermé tel qu'une pièce ou un bâtiment ;
- ladite nappe comprend au moins 5 appareils ;
- lesdits appareils de mesure de pollution particulaire sont des appareils qui procèdent à des mesures selon l'une ou l'autre des techniques suivantes : mesure de la masse, par pesée directe ou indirecte, des particules, mesure optique du faisceau lumineux diffusé par les particules, atténuation du rayonnement bêta ;
- à ladite étape c/, ladite loi mathématique est établie par régression linéaire, par auto-apprentissage, ou par des réseaux neuronaux.
- ladite nappe comporte également au moins un appareil de mesure de l'humidité ambiante. Par ailleurs, l'invention se rapporte à un programme d'ordinateur comportant des instructions adaptées pour l'exécution des étapes du procédé selon l'une des caractéristiques précédentes, lorsqu'il est exécuté par au moins un processeur.

Enfin, elle se rapporte également à un support d'enregistrement lisible sur un ordinateur sur lequel est enregistré un programme d'ordinateur comportant des instructions pour l'exécution des étapes du procédé selon l'une des caractéristiques précédentes.

### BREVE DESCRIPTION DES DESSINS

D'autres caractéristiques et avantages de l'invention apparaîtront à la lecture de la description détaillée qui va suivre. Elle sera faite en référence aux dessins annexés dans lesquels :
La figure 1 est un schéma qui illustre une première étape du procédé selon la présente invention ;

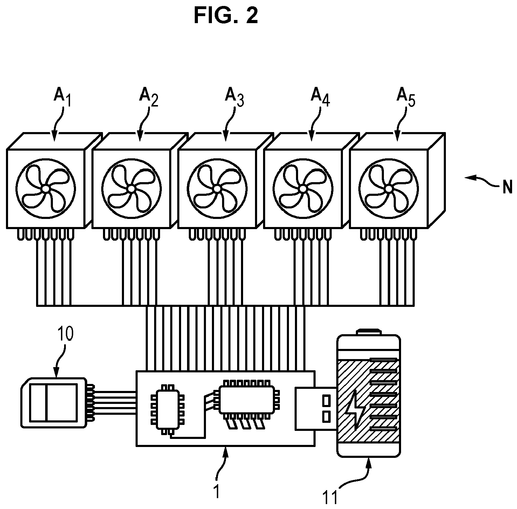

La figure 2 est un schéma qui représente une "nappe" d'appareils de mesure de pollution particulaire ainsi que de moyens qui permettent de collecter les mesures réalisées par ceux-ci ;

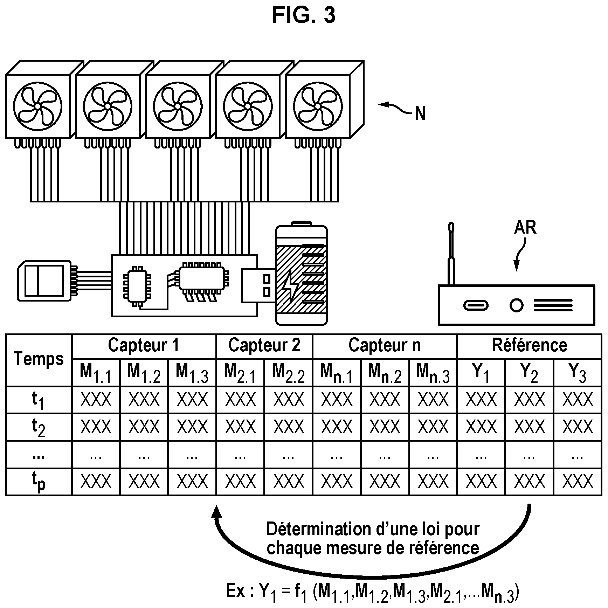

La figure 3 est un schéma destiné à expliquer comment on établit la loi mathématique du procédé selon l'invention ;
La figure 4 est une vue similaire à la figure 1 illustrant la dernière étape du procédé ;

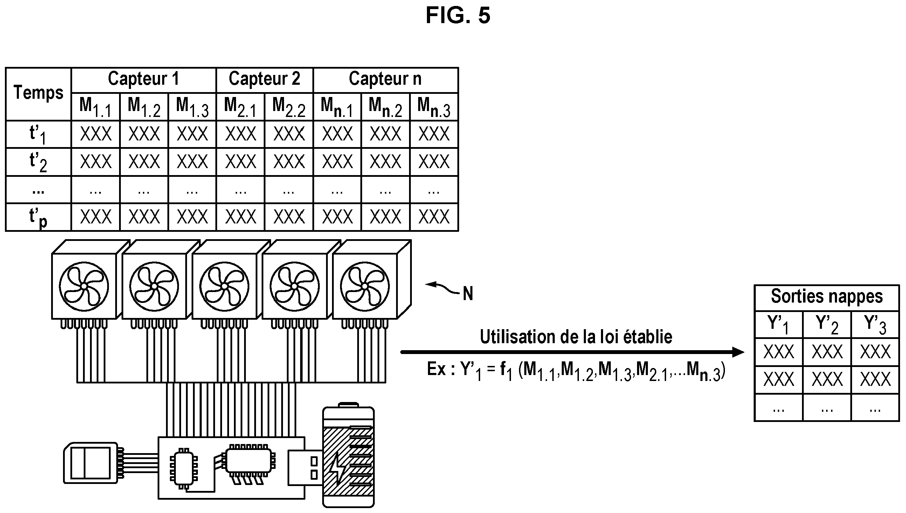

La figure 5 est un schéma similaire à celui de la figure 3 destiné à expliquer comment est mise en œuvre la dernière étape du procédé selon l'invention.

### DESCRIPTION DETAILLEE DE L'INVENTION

L'approche proposée selon la présente invention consiste globalement à associer plusieurs capteurs de particules (appareils de mesure) afin de tirer profit de la différence de leur réponse pour améliorer la qualité des mesures de l'ensemble (appelé « nappe de capteurs » dans la suite). Préférentiellement, ces capteurs sont des capteurs à bas coût de sorte que les mesures effectuées par cette nappe présentent un coût de revient faible par rapport à celles qui seraient effectuées par un appareil de référence plus sophistiqué et plus cher, tel qu'un appareil de laboratoire.

Cette approche exploite des informations supplémentaires venant de la différence de réponse entre les capteurs constitutifs de la nappe, vis-à-vis d'une même pollution.

Malgré le fait que la plupart des micro-capteurs à bas coût commercialisés utilisent la technologie de mesure par diffusion de la lumière, il existe toutefois une diversité de conception (angles de détection, longueur d'onde, configuration du flux d'air, ventilation interne, etc.) pouvant conduire à une diversité de réponses selon la nature des particules.

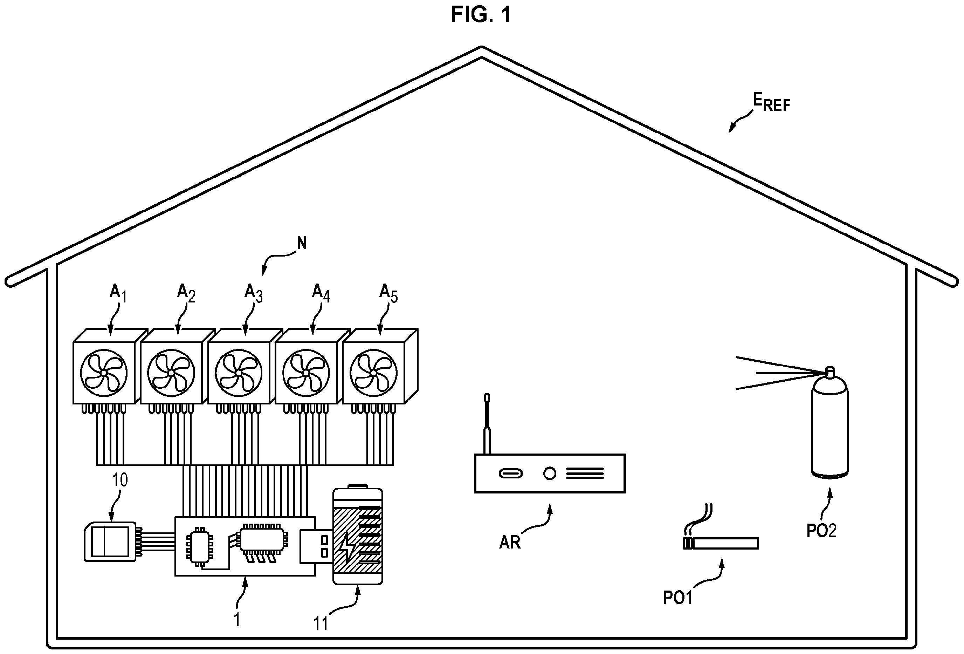

On a représenté à la figure 1 annexée, de manière très schématique, un environnement de référence E_{REF} tel qu'un local fermé (bâtiment, pièce d'un bâtiment, etc.) dans lequel est mis en œuvre une première étape du procédé selon l'invention.

A titre d'exemple, on se propose de mesurer la pollution particulaire générée par deux types de pollution PO1 et PO2, constituées respectivement de fumées de tabac et d'aérosols (par exemple ménagers).

Selon ladite première étape du procédé de l'invention, on dispose dans cet environnement de référence E_{REF}, d'une part un appareil de référence de laboratoire AR de mesure de pollution particulaire (par exemple : Mini Wras de la marque Grimm Aerosol Tecnik) et d'autre part une "nappe" N, c'est à dire un ensemble d'au moins deux autres appareils de mesure de pollution particulaire, les appareils de ladite nappe N étant différents les uns des autres, mais également dudit appareil de référence AR.

En l'occurrence, la nappe N est constituée de cinq appareils référencés A₁ à A₅. Bien entendu, ce nombre peut être inférieur ou supérieur à cinq. Le cas échéant, des capteurs de conditions ambiantes (notamment humidité) peuvent être ajoutés.

Par l'expression, "les appareils de ladite nappe N étant différents les uns des autres", on entend que les appareils se différentient les uns des autres soit par la méthode même de mesure de la pollution, soit, lorsque la méthode de mesure est la même, par au moins un paramétrage ou une caractéristiques de conception différente, telle qu'une circulation d'air différente ou une position différente des récepteurs.

L'appareil de référence est préférentiellement un appareil de laboratoire réputé pour la haute qualité de ses mesures.

Le procédé est poursuivi en collectant, pendant une durée prédéterminée et à plusieurs instants t pendant cette durée, les mesures réalisées par la totalité des appareils précités et en constituant une base de données de l'ensemble de ces mesures. Il peut s'agir des sorties, brutes ou non, de ces appareils.

Pour ce faire, la nappe N est avantageusement connectée à un microprocesseur 1 équipé d'une carte mémoire 10 et d'une alimentation électrique 11.

Cette période, dite "période d'apprentissage" est variable et peut aller d'une journée à plusieurs jours.

L'étape suivante consiste à établir, uniquement à partir des mesures de ladite base de données, une loi mathématique reliant la mesure réalisée par ledit appareil de référence AR à celles de ladite nappe N d'appareils

L'objectif de cette étape est d'établir une loi reliant les données mesurées par l'ensemble des capteurs de la nappe N et celles obtenues par le capteur de référence AR. Cette loi peut être établie par différentes méthodes telles que la régression linéaire multiple, l'auto-apprentissage (méthodes de Lasso, ou Ridge par exemple), les réseaux neuronaux, etc.

A titre d'exemple, une approche statistique par régression linéaire multiple par la méthode des moindres carrés a été utilisée pour établir une fonction de type : mesure de l'appareil de référence AR = f(mesures des appareils de la nappe N). Les mesures de référence sont par exemple des concentrations particulaires (en µg/m³ ou en particules/m³) pour certaines classes granulométriques de particules.

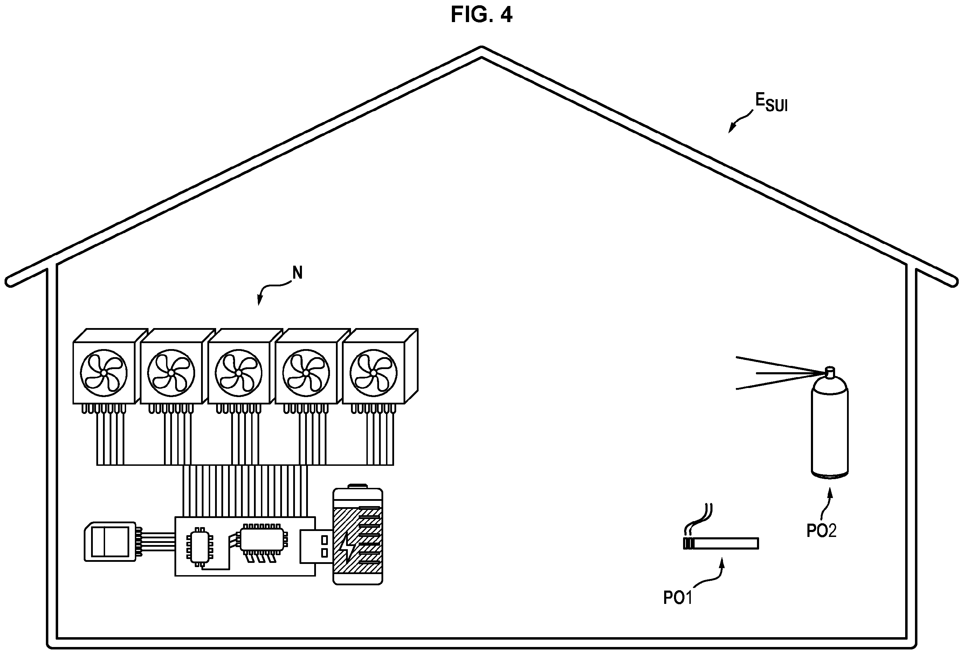

Dans l'étape suivante du procédé, seule la nappe N de d'appareils est placée dans l'environnement suivi E_{SUI}, comme le montre la figure 4.

Bien entendu, les environnements E_{REF} et E_{SUI} sont des environnements de même type, ce qui signifie qu'ils présentent des caractéristiques analogues en termes de configuration et de nature de la pollution.

Ainsi, si l'environnement suivi est une maison dans laquelle résident des fumeurs et où on fait usage d'aérosols ménagers, on choisira un environnement de référence analogue, et non pas une salle de classe où ces deux sources de pollution ne sont pas présentes.

La loi mathématique déterminée précédemment est appliquée à la nappe N d'appareils, afin de « prédire » les mesures que donnerait l'appareil de référence AR, dans un tel environnement. Ces prédictions constituent les mesures communiquées à l'utilisateur par la nappe N.

Des tests de mise en œuvre de ce procédé ont été réalisés.

Les principaux résultats obtenus dans le cadre de ces essais sont les suivants :
1/ Un dispositif expérimental a permis de constituer des jeux d'apprentissage et de tests représentatifs des secteurs résidentiels et tertiaires.
2/ Les jeux de données ainsi générés ont permis de prouver la capacité d'une nappe de capteurs à prédire avec précision les mesures d'un appareil de référence et ceci sur plusieurs scénarios, à savoir un jeu test sur une zone incluse dans la phase d'apprentissage et un jeu test sur une zone non incluse dans la phase d'apprentissage.
   Les résultats obtenus pour la prédiction d'indicateurs classiques de la pollution particulaire (PM1, PM2.5 et PM10) sont encourageants, y compris pour des pollutions de nature très différentes. Le fonctionnement en nappe des capteurs a également permis d'estimer avec une erreur de moins de 25% la concentration moyenne en nanoparticules de diamètres inférieurs à la limite de détection des capteurs à bas coût pris séparément.
3/L'intérêt de la prise en compte simultanée des mesures de plusieurs capteurs a été prouvé ;
4/Le prototype de nappe étudié a permis de lever deux des verrous principaux de la mesure à bas coût des particules pour leur utilisation en QAI, c'est à dire la fiabilité des mesures et la limite de détection.

Le prototype de nappe constitué a pu se baser sur l'association de capteurs fonctionnant sur le même principe de diffusion de la lumière mais avec des conceptions différentes. Il s'agit d'un choix imposé par l'offre commerciale actuelle. L'ajout futur de capteurs compatibles en taille, en fonctionnement et en coût mais fonctionnant sur d'autres principes physiques (par exemple de pesée) est à envisager.

En effet cette méthode repose sur l'information liée à la différence de perception d'une même pollution entre différents capteurs. L'utilisation de principes physiques de mesure différents devrait donc potentiellement améliorer la qualité des mesures. Plusieurs technologies candidates existent (microbalances à quartz, technologie de détection capacitives, etc.).

En outre, l'approche adoptée se base sur les sorties directes des capteurs. Des variables de type rapport ou différences entre les mesures à bas coût, ou encore des fonctions de ces variables (puissances, racine, logarithme, etc.) sont utilisables.

Deux approches sont possibles pour mettre en œuvre le procédé général décrit précédemment.

Celles-ci correspondent à deux domaines d'application différents.

La première consiste à réaliser l'apprentissage au sein même de la zone concernée, pendant une période précédant la phase opérationnelle (où la nappe de capteurs sera seule, sans l'appareil de référence). Après cette période d'apprentissage, la nappe de capteurs peut être laissée seule et l'appareil référence n'est pas immobilisé.

La seconde consiste à concevoir une base de données représentative d'un type d'environnement (par exemple résidentiel) et à déterminer la loi liant les données des micro-capteurs à bas coût et celles de l'appareil de référence. La nappe est ensuite placée dans une zone inconnue mais cohérente avec la base de données (par exemple un appartement habité). Cette approche nécessite la constitution d'une base de données mais les nappes de capteurs sont opérationnelles dès leur installation au sein de l'ambiance à contrôler.

Les utilisateurs potentiels d'un tel procédé sont les suivants :
- Les entreprises réalisant des diagnostics de qualité de l'air intérieur ;
- Les particuliers désireux de connaitre la qualité de l'air dans leur intérieur ;
- Les gestionnaires de bâtiments privés ou publics (ex : écoles, bâtiment de bureaux, ...) désirant contrôler la qualité de l'air ;
- Les concepteurs de système de gestion de la ventilation.

## Revendications

1. Procédé de mesure en continu de la pollution particulaire dans un environnement déterminé, dit "environnement suivi" (Eₛᵤᵢ), **caractérisé par le fait qu'**il comprend les étapes qui consistent à :
a/ disposer dans un environnement de référence (E_{ref}) qui est du même type de ledit environnement suivi (Eₛᵤᵢ), d'une part un appareil de référence (AR) de mesure de pollution particulaire et d'autre part d'une "nappe" (N), c'est à dire un ensemble d'au moins deux autres appareils (A1-A5) de mesure de pollution particulaire, les appareils (A1-A5) de ladite nappe (N) étant différents les uns des autres, mais également dudit appareil de référence (AR) ;
b/ pendant une durée prédéterminée et à plusieurs instants t pendant cette durée, collecter les mesures réalisées par la totalité de ces appareils (A1-A5, AR) et constituer une base de données de l'ensemble de ces mesures ;
c/ établir, uniquement à partir des mesures de ladite base de données, une loi mathématique reliant la mesure réalisée par ledit appareil de référence (AR) à celles de ladite nappe (N) d'appareils ;
d/ disposer, dans ledit environnement suivi (Eₛᵤᵢ), seulement la nappe (N) d'appareils, et procéder à des mesures à l'aide de cette nappe (N) pour prédire, en faisant usage de ladite loi mathématique, les mesures que donnerait ledit appareil de référence (AR) s'il était dans cet environnement suivi (Eₛᵤᵢ).

2. Procédé selon la revendication 1, **caractérisé par le fait que** ledit environnement suivi (Eₛᵤᵢ) est un espace ouvert ou un espace fermé tel qu'une pièce ou un bâtiment.

3. Procédé selon la revendication 1 ou 2, **caractérisé par le fait que** ladite nappe (N) comprend au moins 5 appareils.

4. Procédé selon l'une des revendications précédentes, **caractérisé par le fait que** lesdits appareils de mesure de pollution particulaire sont des appareils qui procèdent à des mesures selon l'une ou l'autre des techniques suivantes : mesure de la masse, par pesée directe ou indirecte, des particules, mesure optique du faisceau lumineux diffusé par les particules, atténuation du rayonnement bêta.

5. Procédé selon l'une des revendications précédentes, **caractérisé par le fait que** à ladite étape c/, ladite loi mathématique est établie par régression linéaire, par auto-apprentissage, ou par des réseaux neuronaux.

6. Procédé selon l'une des revendications précédentes, **caractérisé par le fait que** ladite nappe (N) comporte également au moins un appareil de mesure de l'humidité ambiante.

7. Programme d'ordinateur comportant des instructions adaptées pour l'exécution des étapes du procédé selon l'une des revendications précédentes, lorsqu'il est exécuté par au moins un processeur.

8. Support d'enregistrement lisible sur un ordinateur sur lequel est enregistré un programme d'ordinateur comportant des instructions pour l'exécution des étapes du procédé selon l'une des revendications 1 à 5.
